# EUROPÄISCHE PATENTSCHRIFT

(11) **EP 0 488 943 B1**
(45) Veröffentlichungstag und Bekanntmachung des Hinweises auf die Patenterteilung: **24.08.1994**
(21) Anmeldenummer: 91810826.7
(22) Anmeldetag: 25.10.1991
(51) Int. Cl.: A61F 2/34

(54) **Hüftgelenkschale aus Metall**
Metallic hip joint cup
Cupule métallique pour l'articulation de la hanche

(30) Priorität: 26.11.1990 CH 3737/90
(43) Veröffentlichungstag der Anmeldung: 03.06.1992
(73) Patentinhaber: SULZER Medizinaltechnik AG, 8404 Winterthur (CH); PROTEK AG, 3110 Münsingen-Bern (CH)
(72) Erfinder: Wagner, Heinz, Prof. Dr.-med. Orthopädische, W-8501 Schwarzenbruck (DE); Willi, Roland, CH-8413 Neftenbach (CH)
(74) Vertreter: Trieblnig, Adolf

(56) Entgegenhaltungen:
- DE-A- 3 446 048
- FR-A- 2 615 726
- FR-A- 2 639 822

## Beschreibung

Die Erfindung betrifft eine Hüftgelenkschale aus Metall zum Einsetzen in Knochengewebe bestehend aus zwei durch eine Schnappverbindung in Richtung ihrer Polachsen miteinander verankerten Schalen, von denen die innere Schale aus einer Kobaltlegierung und die äussere Schale aus Titan besteht.

Eine zweiteilige metallische Hüftgelenkspfanne ist in der DE-PS 34 46 048 gezeigt, die eine lösbare Klemmverbindung zwischen zwei Halbschalen aufweist. Beide Halbschalen sind über grössere Flächen mit Knochengewebe in Berührung und sollten daher aus gewebeverträglichem Material bestehen, was einer Einschränkung in der Materialauswahl entspricht. Im weiteren wird eine Primärverankerung mit Tragrippen und mit einer Einschlaghülse gezeigt, die von der Haftung des beim Einschlagen rückfedernden Knochengewebes abhängig ist. Der Zustand des Knochengewebes, der gerade bei älteren Patienten als nicht mehr so elastisch anzusehen ist, entscheidet mit über die Güte der Primärverankerung.

Hier schafft die Erfindung Abhilfe. Sie löst die Aufgabe, eine sichere Primärverankerung vorzunehmen. Gemäss der Erfindung wird die Aufgabe dadurch gelöst, dass die äussere Schale in Richtung der Polachse einen ins Knochengewebe ragenden Spreizdübel aufweist, der in radial auslenkbare Segmente aufgeteilt ist, dass die innere Schale drehfest mit einem Formkörper von unterschiedlichen Durchmessern verbunden ist, der innerhalb des Spreizdübels drehbar ist und der mit seiner Verdrehung gegenüber dem Spreizdübel diesen aufspreizt und in einer Halteposition einrastet, und dass die innere Schale bei eingehängter Schnappverbindung gegenüber der äusseren Schale um die Polachse drehbar ist.

Die Vorteile der Erfindung sind darin zu sehen, dass Verankerungselemente der eingelegten und angepressten Hüftgelenksschale quer zur Einbringrichtung eine vorgegebene Verankerungsbewegung ausführen und in das Knochengewebe eingreifen. Die abhängigen Unteransprüche 2 und 3 beziehen sich auf vorteilhafte Weiterbildungen der Erfindung.

Im folgenden wird die Erfindung anhand eines Ausführungsbeispiels beschrieben. Es zeigen:
- Fig. 1: Schematisch die Seitenansicht einer Hüftgelenkspfanne mit Spreizdübel;
- Fig. 2: schematisch die Draufsicht auf eine Hüftgelenkspfanne gemäss Fig. 1 mit eingerastetem Formkörper und mit gespreizten Verankerungselementen; und
- Fig. 3: schematisch den Schnitt durch eine im Knochengewebe eingesetzte Hüftgelenkspfanne gemäss Fig. 1;
- Fig. 4: einen Ausschnitt gemäss Fig. 2 mit nicht eingerastetem Formkörper und mit ungespreizten Verankerungselementen.

Die Figuren zeigen eine Hüftgelenksschale aus Metall zum Einsetzen in Knochengewebe, bestehend aus zwei durch eine Schnappverbindung in Richtung ihrer Polachsen miteinander verankerten Schalen, von denen die innere Schale aus einer Kobaltlegierung und die äussere Schale aus Titan besteht. Erfindungsgemäss weist die äussere Schale in Richtung der Polachse einen ins Knochengewebe ragenden Spreizdübel auf, welcher in radial auslenkbare Segmente aufgeteilt ist. Die innere Schale ist drehfest mit einem Formkörper von unterschiedlichen Durchmessern verbunden, der innerhalb des Spreizdübels drehbar ist und der mit seiner Verdrehung gegenüber dem Spreizdübel diesen aufspreizt und in eine Halteposition einrastet. Die innere Schale ist bei eingehängter Schnappverbindung gegenüber der äusseren Schale um die Polachse drehbar.

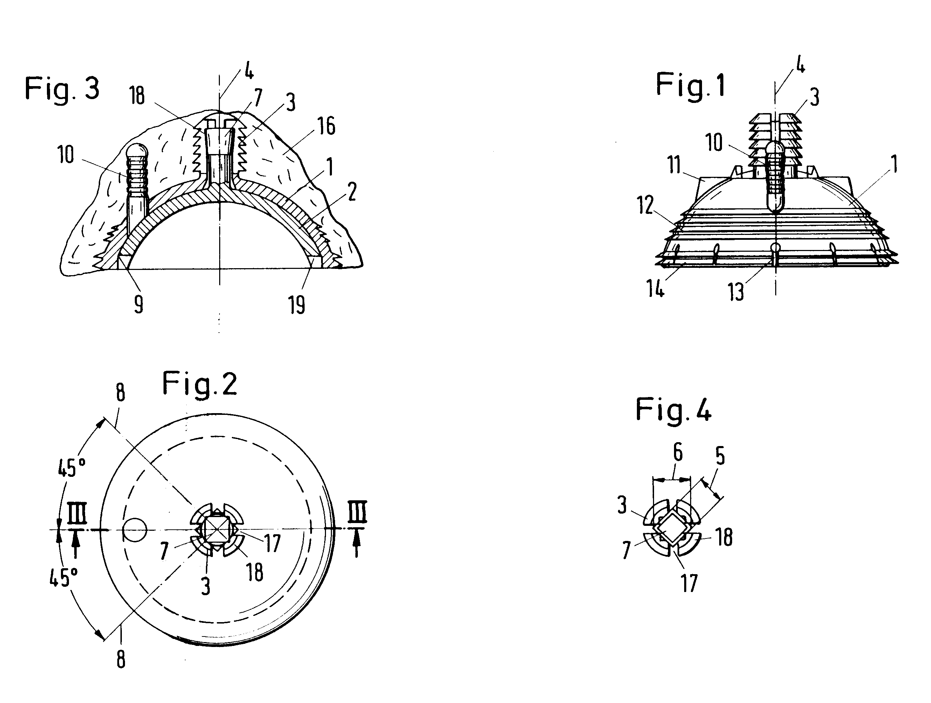

In Fig. 1 ist eine äussere Schale 1 gezeigt, die zur Verankerung im Knochengewebe einen Spreizdübel 3, einen Führungszapfen 10, schaftkantige Dornen 11 und Ringelemente 12 aufweist. Der Spreizdübel 3 steht in Richtung der Polachse 4 von der äusseren Schale 1 weg. Die äusere Schale 1 ist mit Einschnitten 13 versehen, so dass federnde Lappen 14 entstehen, welche beim Einsetzen der inneren Schale 2 in die äussere Schale 1 nach aussen wegfedern können, um die innere Schale 2 mit einer Schnappverbindung 9 in Richtung der Polachse 4 zu sichern.

In Fig. 2 ist der Spreizdübel 3 aufgespreizt worden, indem der Formkörper 7, der in diesem Fall aus einem Vierkant besteht, aus der axialen Einstecklage um 45° gedreht wurde und mit seinen vier Kanten vier Verankerungselemente 18 von der Polachse 4 weg ausgelenkt hat. In Fig. 4 ist der Formkörper 7 in der axialen Einstecklage gezeigt, in der er in den Spreizdübel 3 axial einsteckbar ist. Die den Verankerungselementen 18 gegenüber liegenden Flächen haben einen Abstand 5 zueinander, der wesentlich kleiner ist als der Diagonalabstand 6 der Kanten. Mit der Verdrehung um 45° in Position 8 werden die Verankerungselemente 18 auf Diagonalabstand 6 auseinandergeschoben und die Schlitze 17 zwischen den Verankerungselementen 18 vergrössert. Zur Sicherung gegen unfreiwilliges weiteres Verdrehen sind auf der Innenseite der Verankerungselemente 18 Kerben angebracht, in die die Kanten vom Formkörper 7 einrasten.

In Fig. 3 ist ersichtlich, dass der Formkörper 7 starr mit der inneren Schale 2 verbunden ist, die ihrerseits axial gesichert durch die Schnappverbindung 9 in der äusseren Schale 1 drehbar gelagert ist. Die innere Schale 2 besitzt an ihrem Rand Ausnehmungen 19, an denen die innere Schale 2 mit einem Hilfswerkzeug (hier nicht gezeigt) gegenüber der äusseren Schale 1 verdreht werden kann, um den Spreizdübel 3 aufzuspreizen. Das bei der Verdrehung entstehende Moment wird von der äusseren Schale 1 mit dem Spreizdübel 3 und dem im Abstand dazu plazierten Führungszapfen 10 auf das Knochengewebe 16 übertragen, damit keine zusätzliche Belastung für die im Knochengewebe eingespressten scharfkantigen Dornen 11 und Ringelemente 12 entsteht. Mit dem Aufspreizen des Spreizdübels 3 ist die äussere Schale 1 im Knochengewebe 16 verankert und gesichert. Die Verankerungselemente 18 weisen Vorsprünge auf, die sich mit der Aufspreizbewegung quer zur Polachse 4 in das Knochengewebe 16 eingraben.

Die beiden Schalen 1, 2 werden in vormontiertem Zustand, d.h. mit eingerasteter Schnappverbindung 9, in das vorbearbeitete Knochengewebe 16 in Richtung der Polachse 4 eingepresst und die innere Schale 2 wird gegenüber der äusseren Schale 1 verdreht, bis die Kanten vom Formkörper 7 in Position 8 in Kerben der Verankerungselemente 18 einrasten. Die Verankerungselemente 18 sind zu den Schlitzen 17 hin soweit verrundet, dass sie durch die tragenden Flächen vom Formkörper 7 aufspreizbar sind. Formkörper 7 und die Innenflächen der Verankerungselemente 18 weisen im Eingriffsbereich eine Neigung 15 zur Polachse 4 auf, die beim Aufspreizen des Spreizdübels die innere Schale daran hindert, sich im Rahmen eines möglichen Spiels der Schnappverbindung 9 in axialer Richtung von der äusseren Schale zu lösen.

Durch die Vormontage der beiden Schale 1, 2 ist sichergestellt, dass keine Fremdkörper, wie z.B. Knochenspäne, zwischen die tragenden Schalenflächen gelangen. Gleichzeitig wird eine solide Primärverankerung erreicht, ohne dass die tragende Innenfläche der inneren Schale 2 zum Femurkopf hin Unterbrechungen aufweist, die Schmierfilm und Druckpolster unterbrechen.

## Patentansprüche

1. Hüftgelenkschale aus Metall zum Einsetzen in Knochengewebe bestehend aus zwei durch eine Schnappverbindung (9) in Richtung ihrer Polachsen miteinander verankerten Schalen (1,2), von denen die innere Schale (2) aus einer Kobaltlegierung und die äussere Schale (1) aus Titan besteht, dadurch gekennzeichnet, dass die äussere Schale (1) in Richtung der Polachse (4) einen ins Knochengewebe (16) ragenden Spreizdübel (3) aufweist, der in radial auslenkbare Segmente (18) aufgeteilt ist, dass die innere Schale (2) drehfest mit einem Formkörper (7) von unterschiedlichen Durchmessern (5,6) verbunden ist, der innerhalb des Spreizdübels (3) drehbar ist und der mit seiner Verdrehung gegenüber dem Spreizdübel (3) diesen aufspreizt und in einer Halteposition (8) einrastet, und dass die innere Schale (2) bei eingehängter Schnappverbindung (9) gegenüber der äusseren Schale (1) um die Polachse (4) drehbar ist.

2. Hüftgelenkschale nach Anspruch 1, dadurch gekennzeichnet, dass die äussere Schale (1) wenigstens einen Führungszapfen (10) zum Knochengewebe (16) aufweist, der in der Hauptbelastungsrichtung des Hüftgelenks liegt und der zusammen mit dem Spreizdübel (3) bezüglich Rotation um die Polachse (4) eine Drehsicherung für die äussere Schale (1) bildet.

3. Hüftgelenkschale nach Anspruch 1, dadurch gekennzeichnet, dass die äussere Schale (1) scharfkantige Dornen (11) und Ringelemente (12) zur Verankerung im Knochengewebe (16) aufweist.

4. Hüftgelenkschale nach Anspruch 1, dadurch gekennzeichnet, dass die innere Schale (2) starr mit dem Formkörper (7) verbunden ist, und dass die Eingriffsflächen zwischen Formkörper (7) und Verankerungselemente (18) des Spreizdübels (3) zur Polachse (4) eine gemeinsame Neigung (15) aufweisen.

## Claims

1. A hipjoint socket of metal for insertion in bone tissue, consisting of two cups (1, 2) anchored together in the direction of their polar axes (4) by a snap connection (9), the inner cup (2) consisting of a cobalt alloy and the outer cup (1) of titanium, characterized in that the outer cup (1) exhibits a straddling dowel (3) which projects into the bone tissue (16) in the direction of the polar axis (4) and is subdivided into radially deflectable segments (18), that the inner cup (2) is fixed in rotation to a shaped body (7) which has different diameters (5, 6) and is able to turn inside the straddling dowel (3) and by being twisted with respect to the straddling dowel (3) expands it and snaps into a holding position (8), and that the inner cup (2) with the snap connection (9) engaged is able to turn with respect to the outer cup (1) about the polar axis (4).

2. A hipjoint socket as in Claim 1, characterized in that the outer cup (1) exhibits with respect to the bone tissue (16) at least one guidepin (10) which lies in the direction of the main loading of the hipjoint and forms together with the straddling dowel (3) a security against rotation of the outer cup (1) about the polar axis (4).

3. A hipjoint socket as in Claim 1, characterized in that the outer cup (1) exhibits spikes (11) and annular members (12) with sharp edges for anchoring in the bone tissue (16).

4. A hipjoint socket as in Claim 1, characterized in that the inner cup (2) is connected rigidly to the shaped body (7), and that the areas of engagement between the shaped body (7) and the anchor members (18) of the straddling dowel (3) exhibit a common slant (15) with respect to the polar axis (4).

## Revendications

1. Cavité cotyloïde coxo-fémorale métallique destinée à être implantée dans le tissu osseux et constituée de deux coques (1, 2) qui sont solidarisées l'une à l'autre par une liaison par emboîtement (9) dans le sens de leurs axes polaires et dont la coque intérieure (2) est réalisée en alliage de cobalt et la coque extérieure (1) est réalisée en titane, caractérisée en ce que la coque extérieure (1) comporte, dans le sens de l'axe polaire (4), une cheville expansible (3) qui pénètre dans le tissu osseux (16) et qui est divisée en segments déployables radialement (18), en ce que la coque intérieure (2) est solidaire en rotation avec un corps moulé (7) qui possède différents diamètres (5, 6), qui peut tourner à l'intérieur de la cheville expansible (3) et qui, lors de sa rotation par rapport à la cheville expansible (3), écarte celle-ci et s'encliquette dans une position d'arrêt (8), et en ce que, une fois la liaison par emboîtement (9) réalisée, la coque intérieure (2) peut tourner selon l'axe polaire (4) par rapport à la coque extérieure (1).

2. Cavité cotyloïde coxo-fémorale selon la revendication 1, caractérisée en ce que la coque extérieure (1) comporte au moins un tourillon de guidage (10) qui est implanté dans le tissu osseux (16), qui s'étend dans la direction de sollicitation principale de l'articulation de la hanche et qui forme, conjointement avec la cheville expansible (3), un dispositif de blocage en rotation pour la coque extérieure (1) par rapport à une rotation selon l'axe polaire (4).

3. Cavité cotyloïde coxo-fémorale selon la revendication 1, caractérisée en ce que la coque extérieure (1) comporte des éléments en forme d'épines (11) et des éléments annulaires (12) à arêtes vives destinés à être ancrés dans le tissu osseux (16).

4. Cavité cotyloïde coxo-fémorale selon la revendication 1, caractérisée en ce que la coque intérieure (2) est reliée de manière rigide au corps moulé (7), et en ce que les surfaces de contact entre le corps moulé (7) et les éléments d'ancrage (18) de la cheville expansible (3) présentent une inclinaison commune (15) par rapport à l'axe polaire (4).
